# EUROPEAN PATENT SPECIFICATION

(11) **EP 2 596 753 B1**
(45) Date of publication and mention of the grant of the patent: **10.09.2014**
(21) Application number: 12789423.6
(22) Date of filing: 10.04.2012
(51) Int. Cl.: A61B 8/12, A61B 1/00, G02B 23/24, A61B 5/00, A61B 8/00

(54) **ULTRASOUND ENDOSCOPE**
ULTRASCHALLENDOSKOP
ENDOSCOPE ULTRASONORE

(30) Priority: 20.05.2011 JP 2011113905
(43) Date of publication of application: 29.05.2013
(73) Proprietor: OLYMPUS MEDICAL SYSTEMS CORP., Tokyo 151-0072 (JP)
(72) Inventor: TSURUTA Teppei, Tokyo 151-0072 (JP)
(74) Representative: Schicker, Silvia
(86) International application number: PCT/JP2012/059794
(87) International publication number: WO 2012/160892

(56) References cited:
- JP-A- 7 116 166
- JP-A- 9 238 893
- JP-A- 2001 292 996
- JP-A- 2010 011 989
- US-A1- 2004 082 883
- US-A1- 2008 051 655
- US-B1- 6 461 304

## Description

### Technical Field

The present invention relates to an ultrasound endoscope, a distal end portion of an insertion portion of which is covered by a distal end cover.

### Background Art

In an ultrasound endoscope that can observe an ultrasound image, which is a two-dimensional visible image of a region to be examined, a configuration is well known in which a distal end cover is provided in an outer circumference of a distal end portion made of metal located on a distal end side in an inserting direction of an insertion portion. The configuration is disclosed in, for example, Japanese Patent Application Laid-Open Publication No. 2005-323886.

The distal end cover is made of an insulating member. The distal end cover has a function of covering the outer circumference of the distal end portion to thereby protect the distal end portion besides retaining insulation properties of the distal end portion. However, in the configuration disclosed in Japanese Patent Application Laid-Open Publication No. 2005-323 886, the outer circumference of the distal end portion is covered by the distal end cover but a distal end face of the distal end portion is not covered by the distal end cover.

Note that, on the distal end face, for example, distal end openings for an objective optical system, an illumination optical system, and a treatment instrument insertion channel and an opening of a through-hole are provided. A proximal end portion in an inserting direction of an ultrasound transducer portion provided at the distal end is attached to the opening of the through-hole such that at least a region including a piezoelectric element projects to a front in the inserting direction from the distal end face of the distal end portion.

Therefore, when performing cauterization treatment of a region to be examined by projecting a high-frequency cauterization treatment instrument from the distal end opening of the treatment instrument insertion channel, there has been a case where the distal end face of the distal end portion is cauterized if the high-frequency cauterization treatment instrument comes into contact with the distal end face of the distal end portion which is made of metal directly or via conductive liquid such as water containing impurities.

In view of the above circumstances, it is conceivable to configure the distal end portion by a resin member but in this case it is not preferable since strength of the distal end portion is lowered.

A configuration is naturally conceivable in which a region on the distal end face excluding the openings for the objective optical system, the illumination optical system, and the treatment instrument insertion channel, the opening of the through-hole to which the proximal end portion of the ultrasound transducer portion is attached, and the like is covered using the distal end cover.

Usually, an image pickup unit including the objective optical system, an illumination unit including the illumination optical system, and the like are provided in the distal end portion by being fixed to the distal end portion by screws or the like. If these members arranged in the distal end portion are replaced, when the screws are removed, it is sometimes necessary to detach the distal end cover from the distal end portion in order to expose the screws.

In the configuration in which the distal end cover covers the distal end face of the distal end portion, a distal end side region in the inserting direction of the ultrasound transducer portion is formed larger in an outer diameter according to a shape of the piezoelectric element than a hole formed in the distal end cover that covers the opening to which the proximal end side of the ultrasound transducer portion is attached. Therefore, it is necessary to first detach the ultrasound transducer portion from the distal end portion, and then detach the distal end cover from the distal end portion.

Note that a distal end in an inserting direction of a conductor wire portion for performing at least exchange of electric power and an electric signal with the ultrasound transducer portion is electrically connected to the ultrasound transducer portion. The conductor wire portion is inserted through the insertion portion of the ultrasound endoscope, an operation portion provided at a proximal end in the inserting direction of the insertion portion, a universal cord extending from the operation portion, and a connector provided at an extension end of the universal cord. The conductor wire portion has a predetermined length.

Therefore, when the ultrasound transducer portion is detached from the distal end portion, it is necessary to disconnect a proximal end region in the inserting direction of the conductor wire portion located in the operation portion and in the connector, and then draw out, together with the ultrasound transducer portion, the conductor wire portion further forward in the inserting direction than the distal end face of the distal end portion.

After drawing out the conductive wire portion, in order to detach the distal end cover from the conductor wire portion, there is no choice but to draw out the entire conductor wire portion having predetermined length to the front in the inserting direction via the hole of the distal end cover because a diameter of the hole that covers the opening in which the proximal end portion of the ultrasound transducer portion of the distal end cover is fit is smaller than the outer diameter on the ultrasound transducer portion distal end side.

Besides, the distal end cover can be easily detached from the conductor wire portion by breaking the distal end cover, but there arises a problem that when attaching a new distal end cover to the distal end portion the new distal end cover can not be attached by being obstructed by the conductor wire portion, and therefore the entire conductor wire portion has to be drawn out to the front in the inserting direction for changing the distal end cover, which has been cumbersome for an operator.

Further information pertaining to the prior art can be found in US 2008/051655 that discloses an ultrasonic endoscope that includes an insertion section, an operation section, an optical observation system, and an ultrasonic observation system. The optical observation system is disposed in the insertable section, and has an objective lens located on a face of a distal end of the insertion section. The ultrasonic observing system is disposed in the insertion section, and has an ultrasonic transducer disposed on the face of the distal end of the insertion section or further forward than the distal end.

US patent 6,461,304 discloses an ultrasound inspection apparatus detachably connected to an endoscope comprising an ultrasonic scanning portion in the frontend side having an ultrasound transducer for performing ultrasonic scanning by arranging a number of transducer chips in a rectangular arrangement. The ultrasonic scanning portion is detachably attached to a distal end portion of an insertion unit in the endoscope so as to protrude ahead the distal end portion by a predetermined length. In order to hold the ultrasonic scanning portion in a fixed state, an endoscope-placing portion for placing the distal end portion is connected to the base end of the ultrasonic scanning portion, and an endoscope-fixing portion for detachably fixing the distal end portion thereto is arranged with the endoscope-placing portion. A predetermined number of wires connected to each transducer chip is inserted into a signal cable from a base end position of the endoscope-placing portion so as to bundle the wires.

The present invention has been made in view of the above circumstances, and an object of the present invention is to provide an ultrasound endoscope having a configuration enabling a distal end cover to be easily detachably attached so as to cover a distal end face as well as an outer peripheral surface of a distal end portion.

### Disclosure of Invention

### Means for Solving the Problem

The present invention provides an ultrasound endoscope in accordance with independent claim 1. Preferred embodiments of the invention are reflected in the dependent claims.

### Brief Description of the Drawings

Fig. 1 is an exploded perspective view of a distal end portion in an insertion portion of an ultrasound endoscope according to a first embodiment;
Fig. 2 is a diagram of the distal end portion shown in Fig. 1 schematically viewed from a II direction in Fig. 1 together with a bending portion;
Fig. 3 is a diagram showing a state in which a distal end cover, an air/water feeding nozzle, and an ultrasound transducer unit are attached to a distal end rigid member of the distal end portion shown in Fig. 2;
Fig. 4 is a diagram of the distal end portion shown in Fig. 3 viewed from a IV direction in Fig. 3;
Fig. 5 is a sectional view of the distal end portion taken along a V-V line in Fig. 3;
Fig. 6 is a diagram schematically showing a distal end side of an insertion portion of an ultrasound endoscope according to a second embodiment;
Fig. 7 is a sectional view of a distal end portion taken along a VII-VII line in Fig. 6;
Fig. 8 is an exploded view schematically showing a distal end side of an insertion portion of an ultrasound endoscope according to a third embodiment;
Fig. 9 is a sectional view of an ultrasound transducer unit taken along a IX-IX line in Fig. 8;
Fig. 10 is a sectional view of a distal end portion taken along a X-X line in Fig. 8; and
Fig. 11 is a diagram showing an external appearance of the ultrasound endoscope in which the ultrasound transducer unit is provided in Fig. 1.

### Best Mode for Carrying Out the Invention

Embodiments of the present invention are explained below with reference to the drawings. It should be noted that the drawings are schematic and relations among thicknesses and widths of respective members, ratios of the thicknesses of the respective members, and the like are different from real ones. It goes without saying that portions where relations and ratios of dimensions are different from one another are included among the drawings.

### (First Embodiment)

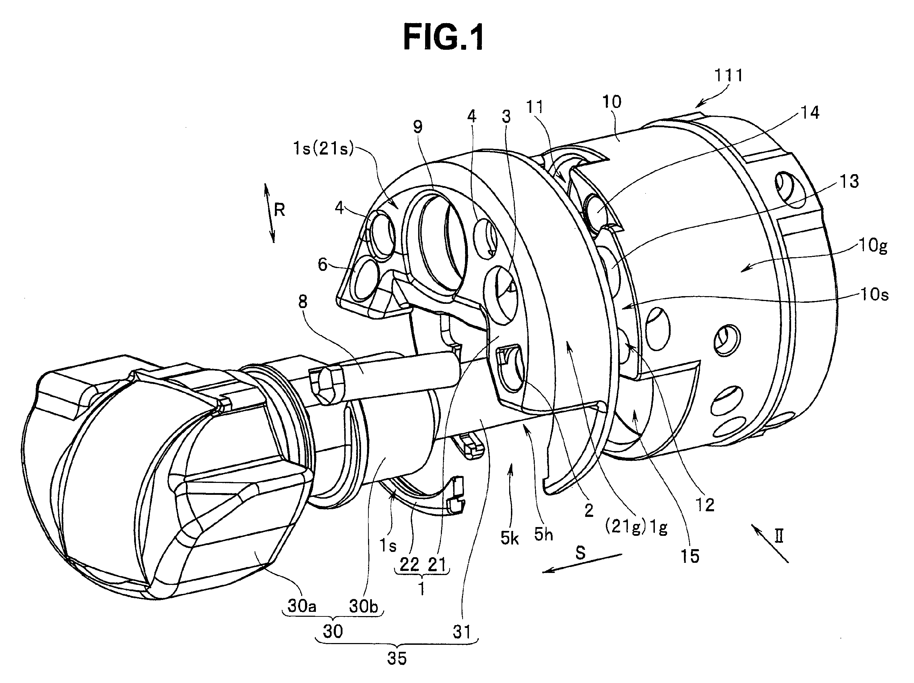

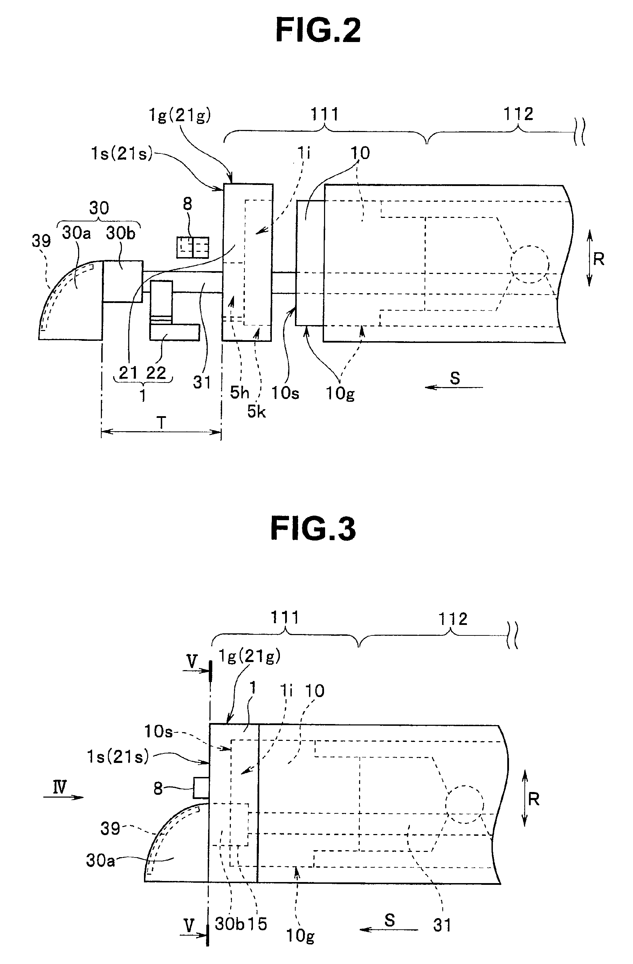

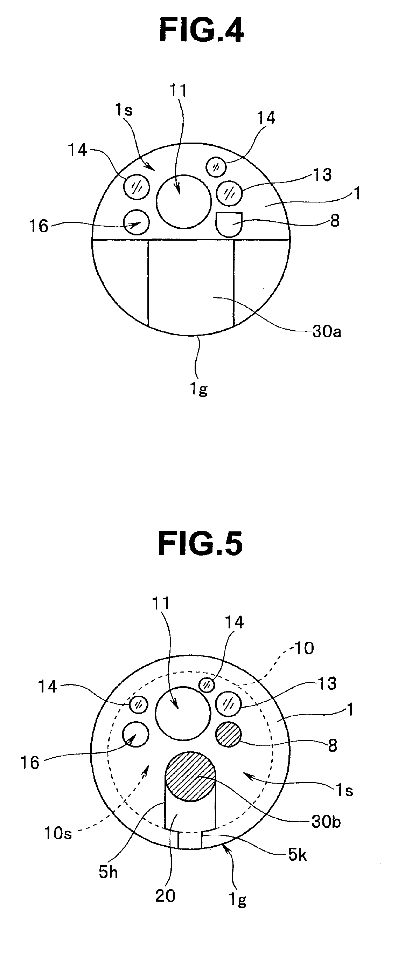

Fig. 1 is an exploded perspective view of a distal end portion in an insertion portion of an ultrasound endoscope according to a first embodiment. Fig. 2 is a diagram of the distal end portion shown in Fig. 1 schematically viewed from a II direction in Fig. 1 together with a bending portion. Fig. 3 is a diagram showing a state in which a distal end cover, an air/water feeding nozzle, and an ultrasound transducer unit are attached to a distal end rigid member of the distal end portion shown in Fig. 2. Fig. 4 is a diagram of the distal end portion shown in Fig. 3 viewed from a IV direction in Fig. 3. Fig. 5 is a sectional view of the distal end portion taken along a V-V line in Fig. 3.

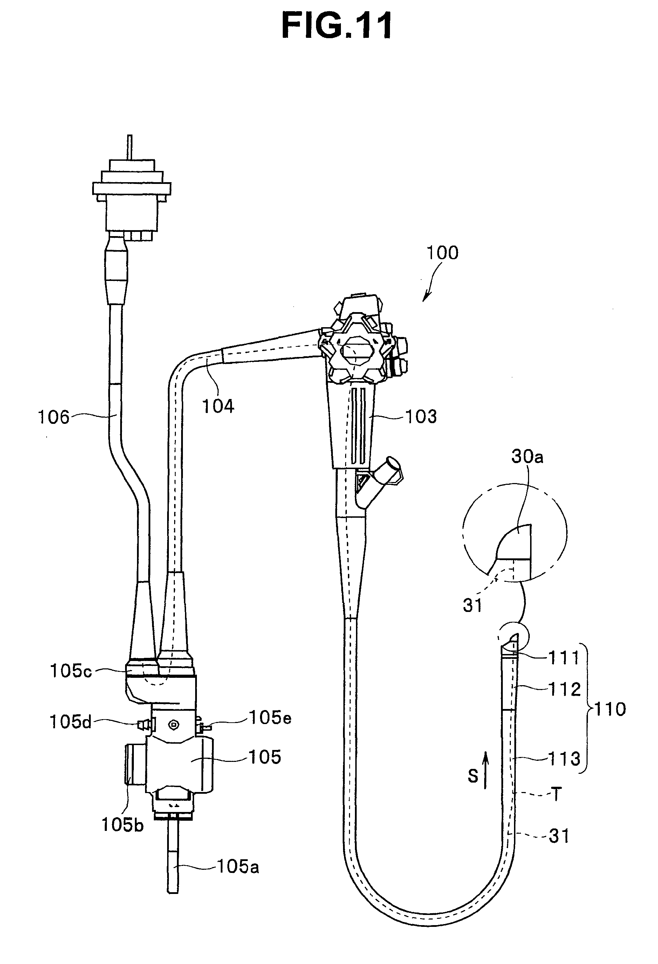

As shown in Figs. 1 to 3, a distal end portion 111 located at a distal end of an inserting direction S in an insertion portion 110, which is inserted into a subject, of an ultrasound endoscope 100 explained below (for the insertion portion 110 and the ultrasound endoscope 100, see Fig. 11) includes a distal end rigid member 10. The distal end rigid member 10 is made of, for example, metal.

In the distal end rigid member 10, a distal end side in the inserting direction S of an ultrasound transducer unit 35, a distal end side in the inserting direction S of a treatment instrument insertion channel 11, an image pickup unit and an illumination unit, a distal end side in the inserting direction of an air/water feeding conduit 12, a distal end side in the inserting direction of a forward water feeding conduit 16 (see Figs. 4 and 5), and the like are provided along the inserting direction S.

Note that the treatment instrument insertion channel 11, the air/water feeding conduit 12, and the forward water feeding conduit 16 are inserted through an operation portion 103, a universal cord 104, and a connector 105 (for all of which, see Fig. 11) besides the insertion portion 110 of the ultrasound endoscope 100.

A main portion of the ultrasound transducer unit 35 includes an ultrasound transducer portion 30 including a first region 30a and a second region 30b and a conductor wire portion 31, one end of which is electrically connected to the ultrasound transducer portion 30. The conductor wire portion 31 performs at least exchange of electric power and an electric signal with the ultrasound transducer portion 30.

The conductor wire portion 31 is inserted through the connector 105, the universal cord 104, the operation portion 103, and the insertion portion 110 of the ultrasound endoscope 100, explained below (for all of which, see Fig. 11). Note that the conductor wire portion 31 is inserted through the insertion portion 110 in a state in which the conductor wire portion 31 is loosened by a predetermined length T, for example, about several centimeters in the insertion portion 110.

The conductor wire portion 31 usually includes a configuration in which a knitted shield formed by knitting metal element wires for improving anti-noise properties is coated on an outer circumference of a plurality of twisted coaxial lines, in which signal lines, GND lines, insulating materials, and skins are coaxially provided, and a skin of resin or the like is coated on an outer circumference of the knitted shield.

However, in the configuration in which the knitted shield is used, although the anti-noise properties are high, there is a drawback in that the knitted shield that expands and contracts or bends following bending and tension applied to the insertion portion 110 tends to be broken.

Therefore, in the present embodiment, a configuration is adopted in which a tape of copper foil or the like for reducing radiation noise from the coaxial lines is spirally wound around an outer circumference from a distal end to a proximal end in the inserting direction of the plurality of twisted coaxial lines and a wire element for shield is wound around an outer circumference from a distal end to a proximal end in the inserting direction of the tape.

With such a configuration, it is possible to maintain anti-noise properties equivalent to the knitted shield. Further, since the copper foil tape has high flexibility, the copper foil tape is capable of extending and contracting. Therefore, it is possible to improve mechanical strength.

This is for preventing a hardened portion from being partially formed in a predetermined place of the knitted shield by friction that occurs among the metal element wires according to the expansion and contraction or the bending of the knitted shield.

When the signal lines of the plurality of coaxial lines in the conductor wire portion 31 are electrically connected to a substrate of the ultrasound transducer unit 35, if the knitted shield is coated, there is a drawback in that it is difficult to expose ends of the signal lines. However, with the present configuration, ends of the copper foil tape and the element wires are easily cut. Therefore, it is possible to easily expose the ends of the signal lines.

The element wires wound around the copper foil tape can be formed thinner in a radial direction R than the knitted shield. Therefore, it is possible to reduce a diameter of the insertion portion 110.

Note that the configuration explained above can be applied not only to the conductor wire portion 31 that performs at least exchange of electric power and an electric signal with the ultrasound transducer portion 30 but also to the signal lines extended from the image pickup unit.

The ultrasound transducer unit 35 is detachably attachable to the distal end rigid member 10. The ultrasound transducer unit 35 is provided such that, after being attached, a distal end in the inserting direction S projects further forward in the inserting direction S than a distal end face 10s of the distal end rigid member 10.

Specifically, as shown in Figs. 3 and 5, in the ultrasound transducer portion 30 provided on the distal end side in the inserting direction S of the ultrasound transducer unit 35, the first region 30a is provided to project further forward in the inserting direction S than the distal end face 10s of the distal end rigid member 10.

Note that, on an inside of the first region 30a covered with a housing, an ultrasound transducer is provided. The ultrasound transducer includes a piezoelectric element 39 having a function of radiating, according to vibration, ultrasound on a region to be examined and receiving a reflected sound wave from the region to be examined and converting the reflected sound wave into an electric signal, a GND electrode and a signal electrode that apply a voltage to the piezoelectric element 39, a lens that collects the ultrasound, an acoustic matching layer, a backing material, a substrate to which the signal electrode is electrically connected and the like.

On an inside of the second region 30b covered with a housing, one ends of the signal lines of the conductor wire portion 31 are electrically connected to the substrate of the ultrasound transducer. In the ultrasound transducer portion 30, the conductor wire portion 31 is electrically connected to the piezoelectric element 39 via the substrate and the signal electrode.

In the distal end rigid member 10, a hole 15 that pierces through the distal end rigid member 10 along the inserting direction S is provided. A proximal end side in the inserting direction S of the second region 30b of the ultrasound transducer portion 30 can be fit in the hole 15. The distal end side in the inserting direction S of the conductor wire portion 31 is pierced through the hole 15.

Note that, the proximal end side in the inserting direction S of the second region 30b is fit in the hole 15 and fixed by screws or the like, whereby the ultrasound transducer unit 35 is attached to the distal end rigid member 10. In other words, the ultrasound transducer unit 35 is detachably attachable to the distal end rigid member 10 by the screws or the like.

The distal end side in the inserting direction S of the treatment instrument insertion channel 11, the image pickup unit and the illumination unit, the distal end side in the inserting direction of the air/water feeding conduit 12, the distal end side in the inserting direction of the forward water feeding conduit 16, and the like are also detachably attachable to the distal end rigid member 10 by screws or the like.

Further, as shown in Fig. 5, on the distal end face 10s of the distal end rigid member 10, a distal end opening of the hole 15, an objective optical system 13 included in the image pickup unit, an illumination optical system 14 included in the illumination unit and configured to illuminate a visual field of the objective optical system 13, a distal end opening of the treatment instrument insertion channel 11, a distal end opening of the air/water feeding conduit 12, a distal end opening of the forward water feeding conduit 16, and the like are provided.

Note that, as shown in Fig. 1, the distal end face 10s has a step shape. The distal end opening of the hole 15 is formed in a position of the distal end face 10s recessed further backward in the inserting direction S than a position where the objective optical system 13 and the distal end opening of the air/water feeding conduit 12 are provided such that, when the proximal end side of the second region 30b of the ultrasound transducer portion 30 is attached to the hole 15, the first region 30a projects further forward in the inserting direction S than the distal end face 10s.

On the distal end face 10s, the illumination optical system 14 and the distal end opening of the forward water feeding conduit 16 are also formed in a position recessed further backward in the inserting direction than the position where the objective optical system 13 and the distal end opening of the air/water feeding conduit 12 are provided.

Further, on the distal end face 10s, the distal end opening of the treatment instrument insertion channel 11 is also formed in a position recessed further backward in the inserting direction than a position where the illumination optical system 14 and the distal end opening of the forward water feeding conduit 16 are provided.

Note that an air/water feeding nozzle 8 for feeding fluid to the objective optical system 13 is detachably attachable to the distal end opening of the air/water feeding conduit 12. After the air/water feeding nozzle 8 is attached to the distal end opening of the air/water feeding conduit 12, as shown in Figs. 3 and 5, the distal end side in the inserting direction S of the air/water feeding nozzle 8 is located to project further forward in the inserting direction S than the distal end face 10s.

As shown in Figs. 1 to 4, a distal end cover 1 that covers an outer circumferential surface 10g and the distal end face 10s of the distal end rigid member 10 is detachably attachable to the distal end rigid member 10 via an adhesive or the like. In other words, the distal end rigid member 10 can be fit in an inside 1i of the distal end cover 1.

Note that the distal end cover 1 is made of resin having insulation properties and chemical resistance, for example, polyphenyl sulfone, polysulfone, or PEEK. A main portion of the distal end cover 1 includes a first distal end cover portion 21 and a second distal end cover portion 22.

As shown in Figs. 1 to 3, the main portion of the distal end cover 1 includes a distal end face 1s serving as a region that covers the distal end face 10s and an outer circumferential surface 1 g serving as a region that covers the outer circumferential surface 10g after the distal end cover 1 is attached to the distal end rigid member 10.

On a distal end face 21s of the first distal end cover portion 21 forming the distal end face 1s, as shown in Fig. 1, through-holes 5h, 3, 4, 9, 2, and 6 along the inserting direction S are respectively formed such that the first region 30a of the ultrasound transducer portion 30, the objective optical system 13, the illumination optical system 14, the distal end opening of the treatment instrument insertion channel 11, the air/water feeding nozzle 8, and the distal end opening of the forward water feeding conduit 16 are exposed from the distal end face 10s of the distal end rigid member 10.

The through-hole 5h exposes the first region 30a of the ultrasound transducer portion 30 from the distal end face 10s. After the proximal end side of the second region 30b of the ultrasound transducer portion 30 is attached to the hole 15 from the front in the inserting direction S via the through-hole 5h, the through-hole 5h is closed by a region on the distal end side of the second region 30b. The through-hole 5h forms a region through which the conductor wire portion 31 pierces when the ultrasound transducer unit 35 is detached from the distal end rigid member 10.

Note that, as explained above, the conductor wire portion 31 is inserted through the insertion portion 110 in the state in which the conductor wire portion 31 is loosened by a predetermined length T, for example, several centimeters in the insertion portion 110. Therefore, when the ultrasound transducer unit 35 is detached from the distal end rigid member 10, the ultrasound transducer portion 30 can project further forward in the inserting direction S than the distal end face 10s of the distal end rigid member 10 by the length of the loosening of the conductor wire portion 31, for example, about several centimeters.

The through-hole 3 exposes the objective optical system 13 from the distal end face 10s. The through-hole 4 exposes the illumination optical system 14 from the distal end face 10s. The through-hole 9 exposes the distal end opening of the treatment instrument insertion channel 11 from the distal end face 10s. The through-hole 2 exposes the air/water feeding nozzle 8 from the distal end face 1 0s. The through-hole 6 exposes the distal end opening of the forward water feeding conduit 16 from the distal end face 10s.

Note that, after being attached to the distal end rigid member 10, the distal end cover 1 is pressed against and bonded to the distal end face 10s by the air/water feeding nozzle 8 and the first region 30a of the ultrasound transducer portion 30.

As shown in Figs. 1 and 2, in the first distal end cover portion 21, a cutout portion 5k is formed. The cutout portion 5k is a conductor wire portion passing portion that causes the through-hole 5h and an outer circumferential surface 21g of the first distal end cover portion 21, which forms the outer circumferential surface 1g of the distal end cover 1, to communicate with each other along a radial direction R of the distal end rigid member 10. In other words, the first distal end cover portion 21 has a shape which is cut out in the radial direction R by the cutout portion 5k to communicate the through-hole 5h with the outside of the distal end cover 1.

The cutout portion 5k forms a region where the conductor wire portion 31 passes in the radial direction R from the through-hole 5h to the outside of the distal end cover 1 in order to detach the first distal end cover portion 21 from the ultrasound transducer unit 35 when the ultrasound transducer unit 35 is detached from the distal end rigid member 10, specifically, when the ultrasound transducer portion 30 is drawn out to the front in the inserting direction S by the length of the loosening of the conductor wire portion 31 from the distal end face 10s via the through-hole 5h.

The second distal end cover portion 22 is detachably attachable to the cutout portion 5k of the first distal end cover portion 21 from, for example, the front in the inserting direction S. After being attached, as shown in Figs. 3 and 5, the second distal end cover portion 22 closes the cutout portion 5k. Note that, although not shown in the figure, the second distal end cover portion 22 includes a slip-off preventing mechanism for preventing the second distal end cover portion 22 from suddenly coming off the cutout portion 5k after being attached.

Next, action of the present embodiment is explained.

When, for example, the image pickup unit provided in the distal end rigid member 10 breaks down and needs to be replaced, firstly, an operator detaches, by releasing the screws or the like, the ultrasound transducer portion 30 fixed to the hole 15 of the distal end rigid member 10. As shown in Fig. 1, the operator draws out the ultrasound transducer portion 30 to the front in the inserting direction S by the length of the loosening of the conductor wire portion 31 in the insertion portion 110. When the ultrasound transducer portion 30 is drawn out, the conductor wire portion 31 passes through the through-hole 5h of the distal end cover 1.

Thereafter, as shown in Fig. 1, the operator detaches the air/water feeding nozzle 8 from the distal end opening of the air/water feeding conduit 12 by drawing out the air/water feeding nozzle 8 to the front in the inserting direction S. Note that, after the ultrasound transducer portion 30 is drawn out and the air/water feeding nozzle 8 is detached, nothing presses the distal end cover 1 against the distal end face 10s of the distal end rigid member 10 to a back in the inserting direction S.

Subsequently, the operator detaches, in the distal end cover 1, the second distal end cover portion 22 from the cutout portion 5k of the first distal end cover portion 21.

Thereafter, the operator releases the bonding of the first distal end cover portion 21 to the distal end rigid member 10 and draws out the first distal end cover portion 21 from the distal end rigid member 10 to the front in the inserting direction S.

Subsequently, the operator moves the conductor wire portion 31 in the radial direction R and removes the conductor wire portion 31 from the through-hole 5h to the outside of the distal end cover 1 via the cutout portion 5k, whereby the distal end cover 1 is detached from the ultrasound transducer unit 35.

When the distal end cover 1 is detached, since the cutout portion 5k is formed in the distal end cover 1, it is possible to easily detach the distal end cover 1 from the conductor wire portion 31 in the radial direction R via the cutout portion 5k without drawing out the entire conductor wire portion 31 to the front in the inserting direction S via the through-hole 5h unlike in the past. Note that, usually, the distal end cover 1 is discarded after being detached. However, the distal end cover 1 may be reused.

Thereafter, the image pickup unit is detached from the distal end rigid member 10 via the screws or the like, replaced or repaired, and attached to the distal end rigid member 10 again.

Subsequently, the operator attaches a new distal end cover 1 via the cutout portion 5k by leading the conductor wire portion 31 into the through-hole 5h of the first distal end cover portion 21 from an outer side in the radial direction R. When the new distal end cover 1 is attached, since the cutout portion 5k is formed, it is possible to easily insert the conductor wire portion 31 through the through-hole 5h of the new distal end cover 1.

Thereafter, in order to prevent the conductor wire portion 31 from coming off the through-hole 5h, the operator closes the cutout portion 5k of the first distal end cover portion 21 with the second distal end cover portion 22 and places the distal end cover 1 over the distal end face 10s and the outer circumferential surface 10g of the distal end rigid member 10 from the front in the inserting direction S and fixes the distal end cover 1 with an adhesive.

Finally, the operator pushes in the second region 30b of the ultrasound transducer portion 30 from the front in the inserting direction S to the back in the inserting direction S, fits the proximal end side of the second region 30b in the hole 15 via the through-hole 5h, and thereafter fixes the proximal end side of the second region 30b with screws or the like.

In this way, the image pickup unit is replaced. The above explanation is not limited to the replacement of the image pickup unit. The same holds true in replacement work for various members provided in the distal end rigid member 10 and also holds true in replacement work for the distal end cover 1 itself.

As explained above, in the present embodiment, the distal end face 10s of the distal end rigid member 10 is also covered by the distal end cover 1.

Consequently, it is possible to prevent, using the distal end cover 1 made of a member having insulation properties, a situation in which, when a high-frequency cauterization treatment instrument is projected to the front in the inserting direction S to perform cauterization treatment of a region to be examined, the high-frequency cauterization treatment instrument comes into contact with the distal end face 10s of the distal end rigid member 10, which is made of metal, directly or via conductive liquid such as water containing impurities, and the distal end face 10s is cauterized. Further, it is possible to prevent, using the distal end cover 1 having chemical resistance, a situation in which a chemical adheres to the distal end face 10s and the distal end face 10s is chemically damaged.

In the present embodiment, the cutout portion 5k of the through-hole 5h is formed in the first distal end cover portion 21 of the distal end cover 1. The cutout portion 5k is closed by the second distal end cover portion 22.

Consequently, when the distal end cover 1 is detached from the distal end rigid member 10, it is possible to easily detach the distal end cover 1 simply by, after drawing out the ultrasound transducer portion 30 of the ultrasound transducer unit 35 to the front in the inserting direction S by the length of the loosening of the conductor wire portion 31 in the insertion portion 110, drawing out the distal end cover 1 from the distal end rigid member 10 to the front in the inserting direction S, thereafter detaching the second distal end cover portion 22 from the cutout portion 5k, and allowing the conductor wire portion 31 inserted through the through-hole 5h of the distal end cover 1 to pass to the outside of the distal end cover 1 via the cutout portion 5k.

When the new distal end cover 1 is attached, it is possible to easily attach the distal end cover 1 simply by leading the conductor wire portion 31 into the through-hole 5h via the cutout portion 5k and, thereafter, after closing the cutout portion 5k with the second distal end cover portion 22, attaching the distal end cover 1 and the ultrasound transducer portion 30 to the distal end rigid member 10.

Therefore, unlike the conventional cases, it is unnecessary to draw out the entire conductor wire portion 31 from the through-hole 5h of the distal end cover 1 from the ultrasound endoscope 100 to the front in the inserting direction S and lead the entire conductor wire portion 31 into the ultrasound endoscope 100 again via the through-hole 5h.

Consequently, it is possible to provide the ultrasound endoscope 100 including the configuration in which the distal end cover 1 that covers the distal end face 10s together with the outer circumferential surface 10g of the distal end rigid member 10 of the distal end portion 111 can be easily attached and detached.

### (Second Embodiment)

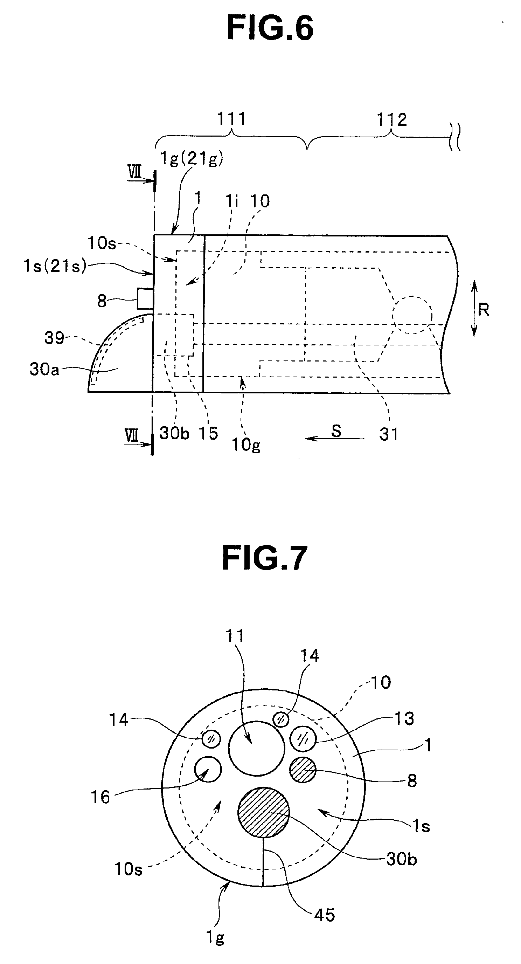

Fig. 6 is a diagram schematically showing a distal end side of an insertion portion of an ultrasound endoscope according to a second embodiment. Fig. 7 is a sectional view of a distal end portion taken along a VII-VII line in Fig. 6.

A configuration of the ultrasound endoscope according to the second embodiment is different from the ultrasound endoscope according to the first embodiment shown in Figs. 1 to 5 in that a distal end cover is formed by one member and a conductor wire portion passing portion provided in the distal end cover is formed by a slit. Therefore, only the difference is explained. Components same as the components in the first embodiment is denoted by the same reference numerals and signs and explanation of the components is omitted.

As shown in Figs. 6 and 7, in the present embodiment, the distal end cover 1 is formed by one member.

In the distal end cover 1, in order to detach the distal end cover 1 from the ultrasound transducer unit 35 when the ultrasound transducer portion 30 is drawn out to the front in the inserting direction S by the length of the loosening of the conductor wire portion 31 from the distal end face 10s via the through-hole 5h, the conductor wire portion passing portion forming the region where the conductor wire portion 31 passes from the through-hole 5h to the outside of the distal end cover 1 is formed by a linear slit 45 formed in the distal end cover 1.

Note that the slit 45 causes the through-hole 5h and the outer circumferential surface 1g of the distal end cover 1 to communicate with each other in the radial direction R. Slit width of the slit 45 is set smaller than a diameter of the conductor wire portion 31.

The slit 45 is a region where the conductor wire portion 31 passes when the conductor wire portion 31 is removed from the through-hole 5h to the outside of the distal end cover 1 or when the conductor wire portion 31 is led into the through-hole 5h from the outside of the distal end cover 1. When the conductor wire portion 31 is removed or when the conductor wire portion 31 is led in, an operator twists and deforms a vicinity of the slit 45 of the distal end cover 1 and increases slit width of the slit 45 to thereby allow the conductor wire portion 31 to pass.

Therefore, the distal end cover 1 in the present embodiment is desirably formed by a member softer than the member forming the distal end cover 1 in the first embodiment. Other components and actions are the same as the components and the actions in the first embodiment explained above.

With such a configuration, since the distal end cover 1 can be formed by one member, it is possible to further reduce manufacturing costs than in the first embodiment. Further, since work for detaching the second distal end cover portion 22 is unnecessary, it is possible to improve workability. Other effects are the same as the effects in the first embodiment explained above.

### (Third Embodiment)

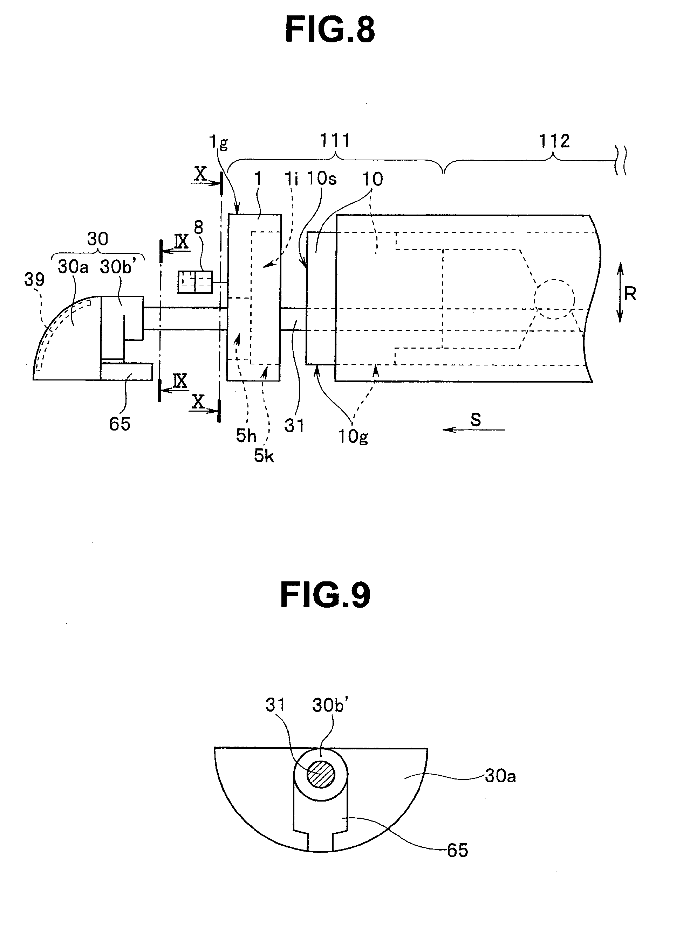

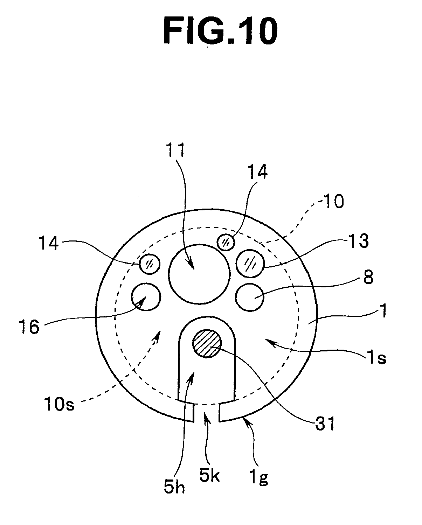

Fig. 8 is an exploded view schematically showing a distal end side of an insertion portion of an ultrasound endoscope according to a third embodiment. Fig. 9 is a sectional view of an ultrasound transducer unit taken along a IX-IX line in Fig. 8. Fig. 10 is a sectional view of a distal end portion taken along a X-X line in Fig. 8.

A configuration of the ultrasound endoscope according to the third embodiment is different from the ultrasound endoscope according to the first embodiment shown in Fig. 1 through Fig. 5 in that a member that closes a cutout portion of a distal end cover is provided in an ultrasound transducer portion. Therefore, only the difference is explained. Components same as the components in the first embodiment is denoted by the same reference numerals and signs and explanation of the components is omitted.

As shown in Figs. 8 and 10, in the present embodiment, as in the first embodiment, in the distal end cover 1, the cutout portion 5k that causes the through-hole 5h and the outer circumferential surface 1g to communicate with each other in the radial direction R is formed. Note that action of the cutout portion 5k in the present embodiment is the same as the action in the first embodiment. Therefore, explanation of the action is omitted.

As shown in Figs. 8 and 9, in a housing of a second region 30b' of the ultrasound transducer portion 30, a closing member 65 that closes the cutout portion 5k when a proximal end side of the second region 30b' of the ultrasound transducer portion 30 is fit in the hole 15 of the distal end rigid member 10 is provided.

Note that the closing member 65 may be formed integrally with the housing of the second region 30b' or may be provided separately from the housing of the second region 30b'.

With such a configuration, as in the first embodiment, the cutout portion 5k is formed simply by drawing out the ultrasound transducer portion 30 from the distal end rigid member 10 to the front in the inserting direction S. Therefore, after the distal end cover 1 is drawn out to the front in the inserting direction S, as in the first embodiment, it is possible to easily remove the conductor wire portion 31 from or lead the conductor wire portion 31 into the through-hole 5h via the cutout portion 5k.

Therefore, even if the distal end cover 1 is formed by one member, it is possible to surely close the cutout portion 5k with the closing member 65 of the ultrasound transducer portion 30 or open the cutout portion 5k. Therefore, it is possible to further reduce manufacturing costs than in the first embodiment.

In the first embodiment, in order to surely attach the second distal end cover portion 22 to the cutout portion 5k of the first distal end cover portion 21, the distal end cover 1 needs to be formed by a member that is less easily plastically deformed. However, in the present embodiment, the cutout portion 5k is closed by the closing member 65 of the ultrasound transducer portion 30. Therefore, it is unnecessary to limit the member forming the distal end cover 1 to the member that is less easily plastically deformed.

Therefore, since a degree of freedom of a material forming the distal end cover 1 increases, it is possible to adopt a material attaching importance to cost and chemical resistance. Other effects are the same as the effects in the first embodiment explained above.

The ultrasound transducer unit 35 explained above is provided in, for example, the ultrasound endoscope 100. A configuration of an ultrasound endoscope in which the ultrasound transducer unit 35 is provided is explained with reference to Fig. 11.

Fig. 11 is a diagram showing an external appearance of an ultrasound endoscope in which the ultrasound transducer unit shown in Fig. 1 is provided.

A main portion of the ultrasound endoscope 100 includes the elongated insertion portion 110 inserted into a subject, the operation portion 103 provided at the proximal end in the inserting direction S of the insertion portion 110, the universal cord 104 having flexibility extended from the operation portion 103, and the connector 105 provided at an extension end of the universal cord 104.

In the connector 105, a light source connector 105a, an electrical connector 105b, an ultrasound connector 105c, a suction pipe sleeve 105d, and an air/water feeding pipe sleeve 105e are provided.

A light source device that supplies illumination light is detachably attachable to the light source connector 105a. A video processor that performs various kinds of signal processing is detachably attachable to the electrical connector 105b via a signal cable.

An ultrasound observation device is detachably attachable to the ultrasound connector 105c via an ultrasound cable 106 connected to the ultrasound observation device. A suction pump is detachably attachable to the suction pipe sleeve 105d via a suction tube. Further, a water feeding tank is detachably attachable to the air/water feeding pipe sleeve 105e via an air/water feeding tube.

The insertion portion 110 is configured by jointly providing, in order from the distal end side in the inserting direction S, the distal end portion 111, a bending portion 112 formed to be capable of bending in, for example, an up-down direction and a left-right direction, and an elongated flexible tube portion 113 having flexibility.

In the distal end portion 111, the conductor wire portion 31 extended from the ultrasound transducer portion 30 is inserted through to the insertion portion 110, the operation portion 103, the universal cord 104, and the ultrasound connector 105c of the connector 105. In the ultrasound connector 105c, the conductor wire portion 31 is electrically connected to the ultrasound cable 106. As explained above, the conductor wire portion 31 is located while being loosened by the predetermined length T in the insertion portion 110.

The configuration of the ultrasound endoscope 100 shown in Fig. 11 above is only an example. The ultrasound endoscope 100 is not limited to the configuration.

## Claims

1. An ultrasound endoscope (100) comprising:
an insertion portion (110) configured to be inserted into a subject;
an objective optical system (13);
an illumination optical system (14) configured to illuminate a visual field of the objective optical system;
a distal end portion (111) located on a distal end side in an inserting direction (S) of an insertion portion inserted into a subject, at least the objective optical system and the illumination optical system being fixed to the distal end portion;
an ultrasound transducer portion (30) detachably attachable to the distal end portion, at least a region of the ultrasound transducer portion including a piezoelectric element (39) projecting further forward in the inserting direction than a distal end face (10s) of the distal end portion;
a distal end cover (1) detachably attachable to the distal end portion, the objective optical system and the illumination optical system which are included in the distal end portion, the distal end cover covering an outer circumferential surface (10g) and the distal end face of the distal end portion such that at least the objective optical system, the illumination optical system, and a region including the piezoelectric element of the ultrasound transducer portion are exposed from the distal end face of the distal end portion, a direction of attachment and detachment of the distal end cover is a direction intersecting with the inserting direction;
a through-hole (5h) formed in the distal end cover to pierce through the distal end cover along the inserting direction and closed by a part of the ultrasound transducer portion when the ultrasound transducer portion is attached to the distal end portion;
a conductor wire portion (31), one end of which is electrically connected to the piezoelectric element, the conductor wire portion piercing through inside of the distal end portion and inside of the insertion portion in the inserting direction and piercing through the through-hole when the ultrasound transducer portion is detached from the distal end portion; and
a conductor wire portion passing portion (5k) formed in the distal end cover and configured to communicate with the through-hole and reach an outer circumference of the distal end cover, the conductor wire portion passing portion forming a region where the conductor wire portion passes from the through-hole to an outside of the distal end cover when the ultrasound transducer portion is detached from the distal end portion.

2. The ultrasound endoscope according to claim 1, wherein the conductor wire portion passing portion is a cutout portion (5k) that communicates with the through-hole formed in the distal end cover.

3. The ultrasound endoscope according to claim 2, wherein the distal end cover includes:
a first distal end cover portion (21) in which the through-hole and the cutout portion are formed; and
a second distal end cover portion (22) detachably attachable to the cutout portion and configured to close the cutout portion after being attached to the cutout portion.

4. The ultrasound endoscope according to claim 2, wherein the ultrasound transducer portion includes a closing member (65) configured to close the cutout portion.

5. The ultrasound endoscope according to claim 4, wherein the closing member is formed integrally with the ultrasound transducer portion.

6. The ultrasound endoscope according to claim 1, wherein the conductor wire portion passing portion is a slit (45), one end of which communicates with the through-hole formed in the distal end cover and the other end of which reaches the outer circumference of the distal end cover.

7. The ultrasound endoscope according to claim 1, wherein
the conductor wire portion is insertable through the insertion portion in a state in which the conductor wire portion is loosened by a predetermined length in the insertion portion, and
when the ultrasound transducer portion is detached from the distal end portion, the ultrasound transducer portion is projectable further forward than the distal end face of the distal end portion by the length of the loosening of the conductor wire portion in the insertion portion.

8. The ultrasound endoscope according to claim 1, wherein a size of the through-hole is smaller than a largest cross section among cross sections in a surface direction of the ultrasound transducer portion.

## Patentansprüche

1. Ultraschallendoskop (100), das umfasst:
einen Einführabschnitt (110), der dazu eingerichtet ist, in ein Subjekt eingeführt zu werden;
ein optisches Objektivsystem (13);
ein optisches Beleuchtungssystem (14), das dazu eingerichtet ist, ein Sichtfeld des optischen Objektivsystems zu beleuchten;
einen distalen Endabschnitt (111), der in einer Einführrichtung (S) eines in ein Subjekt eingeführten Einführabschnitts an einer distalen Endseite angeordnet ist, wobei mindestens das optische Objektivsystem und das optische Beleuchtungssystem an dem distalen Endabschnitt angebracht sind;
einen Ultraschallwandlerabschnitt (30), der lösbar an dem distalen Endabschnitt angebracht ist, wobei mindestens ein Bereich des Ultraschallwandlerabschnitts ein piezoelektrisches Element (39) umfasst, das weiter in die Einführrichtung vorsteht als eine distale Endfläche (10s) des distalen Endabschnitts;
eine distale Endabdeckung (1), die lösbar an dem distalen Endabschnitt, dem optischen Objektivsystem und dem optischen Beleuchtungssystem, die in dem distalen Endabschnitt enthalten sind, angebracht ist, wobei die distale Endabdeckung eine Außenumfangsfläche (10g) und die distale Endfläche des distalen Endabschnitts so abdeckt, dass mindestens das optische Objektivsystem, das optische Beleuchtungssystem und ein Bereich, der das piezoelektrische Element des Ultraschallwandlerabschnitts umfasst, von der distalen Endfläche des distalen Endabschnitts freigelegt sind, und wobei eine Befestigungs- und Löserichtung der distalen Endabdeckung eine Richtung ist, die die Einführrichtung schneidet;
eine Durchgangsöffnung (5h), die in der distalen Endabdeckung ausgebildet ist, um die distale Endabdeckung entlang der Einführrichtung zu durchdringen, und durch ein Teil des Ultraschallwandlerabschnitts geschlossen wird, wenn der Ultraschallwandlerabschnitt mit dem distalen Endabschnitt verbunden ist;
einen Leitungsdrahtabschnitt (31), von dem ein Ende elektrisch mit dem piezoelektrischen Element verbunden ist, wobei der Leitungsdrahtabschnitt ein Inneres des distalen Endabschnitts und ein Inneres des Einführabschnitts in der Einführrichtung durchdringt und die Durchgangsöffnung durchdringt, wenn der Ultraschallwandlerabschnitt von dem distalen Endabschnitt gelöst ist; und
einen Abschnitt (5k) zum Hindurchführen des Leitungsdrahtabschnitts, der in der distalen Endabdeckung ausgebildet ist und dazu eingerichtet ist, mit der Durchgangsöffnung zu kommunizieren und einen Außenumfang der distalen Endabdeckung zu erreichen, wobei der Abschnitt zum Hindurchführen des Leitungsdrahtabschnitts einen Bereich bildet, wo der Leitungsdrahtabschnitt durch die Durchgangsöffnung zu einer Außenseite der distalen Endabdeckung geführt wird, wenn der Ultraschallwandlerabschnitt von dem distalen Endabschnitt gelöst ist.

2. Ultraschallendoskop gemäß Anspruch 1, wobei der Abschnitt zum Hindurchführen des Leitungsdrahtabschnitts ein ausgeschnittener Abschnitt (5k) ist, der mit der Durchgangsöffnung kommuniziert, die in der distalen Endabdeckung ausgebildet ist.

3. Ultraschallendoskop gemäß Anspruch 2, wobei die distale Endabdeckung umfasst:
einen ersten distalen Endabdeckungsabschnitt (21), in dem die Durchgangsöffnung und der ausgeschnittene Abschnitt ausgebildet sind; und
einen zweiten distalen Endabdeckungsabschnitt (22), der lösbar mit dem ausgeschnittenen Abschnitt verbunden ist und dazu eingerichtet ist, den ausgeschnittenen Abschnitt zu verschließen, nachdem er an dem ausgeschnittenen Abschnitt angebracht worden ist.

4. Ultraschallendoskop gemäß Anspruch 2, wobei der Ultraschallwandlerabschnitt ein Schließelement (65) umfasst, das dazu eingerichtet ist, den ausgeschnittenen Abschnitt zu verschließen.

5. Ultraschallendoskop gemäß Anspruch 4, wobei das Schließelement integriert mit dem Ultraschallwandlerabschnitt ausgebildet ist.

6. Ultraschallendoskop gemäß Anspruch 1, wobei der Abschnitt zum Hindurchführen des Leitungsdrahtabschnitts ein Schlitz (45) ist, von dem ein Ende mit der Durchgangsöffnung kommuniziert, die in der distalen Endabdeckung ausgebildet ist, und dessen anderes Ende den Außenumfang der distalen Endabdeckung erreicht.

7. Ultraschallendoskop gemäß Anspruch 1, wobei
der Leitungsdrahtabschnitt in einem Zustand, in dem der Leitungsdrahtabschnitt um eine vorbestimmte Länge in dem Einführabschnitt gelöst ist, durch den Einführabschnitt einführbar ist, und
wenn der Ultraschallwandlerabschnitt von dem distalen Endabschnitt gelöst ist, der Ultraschallwandlerabschnitt um die Löselänge des Leitungsdrahtabschnitts in dem Einführabschnitt weiter vorstehen kann als die distale Endfläche des distalen Endabschnitts.

8. Ultraschallendoskop gemäß Anspruch 1, wobei eine Größe der Durchgangsöffnung kleiner als ein größter Querschnitt unter Querschnitten in einer Oberflächenrichtung des Ultraschallwandlerabschnitts ist.

## Revendications

1. Endoscope (100) à ultrasons comprenant :
une partie d'insertion (110) configurée pour être insérée dans un sujet ;
un système optique d'objectif (13) ;
un système optique d'éclairage (14) configuré pour éclairer un champ visuel du système optique d'objectif ;
une partie d'extrémité distale (111) placée sur un côté d'extrémité distale dans une direction d'insertion (S) d'une partie d'insertion insérée dans un sujet, au moins le système optique d'objectif et le système optique d'éclairage étant fixés à la partie d'extrémité distale ;
une partie de transducteur ultrasonore (30) pouvant être attachée de manière détachable à la partie d'extrémité distale, au moins une région de la partie de transducteur ultrasonore comprenant un élément piézoélectrique (39) faisant saillie davantage vers l'avant dans la direction d'insertion qu'une face d'extrémité distale (10s) de la partie d'extrémité distale ;
un couvercle d'extrémité distale (1) pouvant être attaché de manière détachable à la partie d'extrémité distale, au système optique d'objectif et au système optique d'éclairage qui sont contenus dans la partie d'extrémité distale, le couvercle d'extrémité distale couvrant une surface circonférentielle externe (10g) et la face d'extrémité distale de la partie d'extrémité distale d'une manière telle qu'au moins le système optique d'objectif, le système optique d'éclairage et une région comprenant l'élément piézoélectrique de la partie de transducteur ultrasonore sont exposés à partir de la face d'extrémité distale de la partie d'extrémité distale, une direction de fixation et de séparation du couvercle d'extrémité distale correspondant à une direction croisant la direction d'insertion ;
un trou traversant (5h) formé dans le couvercle d'extrémité distale pour percer à travers le couvercle d'extrémité distale le long de la direction d'insertion et fermé par une partie de la partie de transducteur ultrasonore lorsque la partie de transducteur ultrasonore est attachée à la partie d'extrémité distale ;
une partie de fil conducteur (31) dont une extrémité est électriquement connectée à l'élément piézoélectrique, la partie de fil conducteur perçant à travers l'intérieur de la partie d'extrémité distale et à l'intérieur de la partie d'insertion dans la direction d'insertion et perçant à travers le trou traversant lorsque la partie de transducteur ultrasonore est détachée de la partie d'extrémité distale ; et
une partie (5k) de passage de partie de fil conducteur formée dans le couvercle d'extrémité distale et configurée pour communiquer avec le trou traversant et atteindre une circonférence externe du couvercle d'extrémité distale, la partie de passage de partie de fil conducteur formant une région dans laquelle la partie de fil conducteur passe du trou traversant à un extérieur du couvercle d'extrémité distale lorsque la partie de transducteur ultrasonore est détachée de la partie d'extrémité distale.

2. Endoscope à ultrasons selon la revendication 1, dans lequel la partie de passage de partie de fil conducteur est une partie de découpe (5k) qui communique avec le trou traversant formé dans le couvercle d'extrémité distale.

3. Endoscope à ultrasons selon la revendication 2, dans lequel le couvercle d'extrémité distale comprend :
une première partie (21) de couvercle d'extrémité distale dans laquelle le trou traversant et la partie de découpe sont formés ; et
une deuxième partie (22) de couvercle d'extrémité distale pouvant être attachée de manière détachable à la partie de découpe et configurée pour fermer la partie de découpe après avoir été attachée à la partie de découpe.

4. Endoscope à ultrasons selon la revendication 2, dans lequel la partie de transducteur ultrasonore comprend un élément de fermeture (65) configuré pour fermer la partie de découpe.

5. Endoscope à ultrasons selon la revendication 4, dans lequel l'élément de fermeture est formé solidairement avec la partie de transducteur ultrasonore.

6. Endoscope à ultrasons selon la revendication 1, dans lequel la partie de passage de partie de fil conducteur est une fente (45), dont une extrémité communique avec le trou traversant formé dans le couvercle d'extrémité distale et dont l'autre extrémité atteint la circonférence externe du couvercle d'extrémité distale.

7. Endoscope à ultrasons selon la revendication 1, dans lequel
la partie de fil conducteur peut être insérée à travers la partie d'insertion dans un état dans lequel la partie de fil conducteur est relâchée d'une longueur prédéterminée dans la partie d'insertion, et
lorsque la partie de transducteur ultrasonore est détachée de la partie d'extrémité distale, la partie de transducteur ultrasonore peut faire saillie davantage vers l'avant que la face d'extrémité distale de la partie d'extrémité distale de la longueur de relâchement de la partie de fil conducteur dans la partie d'insertion.

8. Endoscope à ultrasons selon la revendication 1, dans lequel une dimension du trou traversant est plus petite que la section transversale la plus grande parmi les sections transversales dans une direction de surface de la partie de transducteur ultrasonore.
